# EUROPEAN PATENT SPECIFICATION

(11) **EP 4 289 418 B1**
(45) Date of publication and mention of the grant of the patent: **25.03.2026**
(21) Application number: 22750072.5
(22) Date of filing: 07.02.2022
(51) Int. Cl.: A61K 9/19, A61K 47/64, A61K 47/42, A61K 47/26, A61K 47/40, A61K 38/48, A61P 5/50

(54) **BOTULINUM TOXIN FREEZE-DRIED DOSAGE FORM COMPOSITION STORABLE FOR LONG TIME**
GEFRIERGETROCKNETE DARREICHUNGSFORM VON BOTULINUMTOXIN ZUR LAGERUNG ÜBER LANGE ZEIT
COMPOSITION DE FORME POSOLOGIQUE LYOPHILISÉE DE TOXINE BOTULIQUE STOCKABLE PENDANT UNE LONGUE DURÉE

(30) Priority: 08.02.2021 KR 20210017390
(43) Date of publication of application: 13.12.2023
(73) Proprietor: Daewoong Co., Ltd., Seongnam-si, Gyeonggi-do 13211 (KR)
(72) Inventor: SEO, Jeongdeok, Yongin-si, Gyeonggi-do 17074 (KR); KIM, Han-Byul, Yongin-si, Gyeonggi-do 17028 (KR); KIM, Kyoung-Yun, Suwon-si, Gyeonggi-do 16700 (KR)
(74) Representative: Mewburn Ellis LLP
(86) International application number: PCT/KR2022/001824
(87) International publication number: WO 2022/169323

(56) References cited:
- CN-A- 107 540 732
- KR-A- 20080 072 717
- KR-A- 20120 112 248
- KR-A- 20170 030 176
- US-A1- 2005 163 809
- US-A1- 2014 086 900
- US-A1- 2020 215 357
- DRESSLER DIRK ED - HALLETT MARK ET AL: "Therapeutically relevant features of botulinum toxin drugs", TOXICON, ELMSFORD, NY, US, vol. 175, 7 December 2019 (2019-12-07), pages 64 - 68, XP086030149, ISSN: 0041-0101, [retrieved on 20191207], DOI: 10.1016/J.TOXICON.2019.12.005
- DRESSLER D: "Pharmacology of botulinum toxin drugs ; Pharmakologie der Botulinumtoxinmedikamente", HNO ; DEUTSCHE GESELLSCHAFT F�R HALS-NASEN-OHREN-HEILKUNDE, KOPFUND HALS-CHIRURGIE, SPRINGER, BERLIN, DE, vol. 60, no. 6, 7 June 2012 (2012-06-07), pages 496 - 504, XP035066283, ISSN: 1433-0458, DOI: 10.1007/S00106-012-2494-1

## Description

### [Technical Field]

The present invention relates to a botulinum toxin freeze-dried dosage form composition capable of long-term storage, and more particularly to a botulinum toxin freeze-dried dosage form composition capable of long-term storage including a complex of botulinum toxin and protamine sulfate, an isotonic agent, and a buffering agent.

### [Background Art]

Various strains of the genus Clostridium that secrete toxins with neurotoxicity have been discovered since the 1890s and characterization of toxins secreted by these strains has been achieved over the past 70 years.

Toxins with neurotoxicity derived from the strains of the genus Clostridium, namely botulinum toxins, are classified into seven types of A to G depending on serological characteristics thereof. Each toxin has a toxin protein of about 150 kDa and is naturally composed of a complex incorporating various non-toxic proteins. A medium complex (300 kDa) is composed of toxin protein and non-toxic non-hemagglutinin protein, and a large (450 kDa) complex and a large-large (900 kDa) complex are configured such that a medium complex binds to hemagglutinin (Sugiyama, H, Microbiol Rev., 44:419, 1980). These non-toxic non-hemagglutinin proteins are known to function to protect toxins from low pH and various proteolytic enzymes in the intestine.

The toxin is synthesized into a single polypeptide having a molecular weight of about 150 kDa in cells, and then cleaved at a position 1/3 from the N-terminus by the action of an intracellular proteolytic enzyme or artificial treatment with an enzyme, such as trypsin, and thus is divided into two units, namely a light chain (L: molecular weight: 50 kDa) and a heavy chain (H: molecular weight: 100 kDa). The toxicity of the toxin thus divided is greatly increased compared to that of a single polypeptide. The two units are linked to each other by a disulfide bond, and each has a different function. The heavy chain binds to the receptor of a target cell and reacts with a biomembrane at a low pH (pH 4) to form a channel (Mantecucco, C et al, TIBS, 18:324, 1993), and the light chain has pharmacological activity, thus imparting cell penetration activity using a detergent or interfering with the secretion of neurotransmitters when injected into cells by electroporation, etc.

The toxin inhibits the exocytosis of acetylcholine at the cholinergic presynapse of the neuromuscular junction, causing generalized asthenia. It has been thought that this toxin has any enzymatic activity because toxicity appears even when used in a very small amount.

As recently discovered, toxins have metallopeptidase activity, and substrates thereof are synaptobrevin, syntaxin, and synaptosomal-associated protein of 25 kDa (SNAP25), which are unit proteins of the exocytosis machinery complex. Each type of toxin uses one of these three proteins as a substrate. It is known that types B, D, F, and G, types A and E, and type C cleave synaptobrevin, SNAP25, and syntaxin, respectively, at specific sites.

In particular, botulinum toxin type A is known to be soluble in a dilute aqueous solution at a pH of 4.0 to 6.8. It is known that, at a pH of about 7 or higher, the stabilizing non-toxin protein is separated from the neurotoxin, resulting in a gradual loss of toxicity, particularly decreasing toxicity with increasing pH and temperature.

Since the botulinum toxin is fatal to the human body in small amounts and is easy to mass-produce, it is a toxin that may be used for four major bioterrorism weapons along with *Bacillus anthracis, Yersinia pestis,* and smallpox virus. In particular, however, it has been found that botulinum toxin type A may paralyze the local muscles at the injection site when injected at a level equal to or less than a dose that does not affect the human body systemically. Using these characteristics, botulinum toxin type A may serve as a wrinkle remover and a therapeutic agent for spastic hemiplegia and cerebral palsy, and thus the demand therefor is rapidly increasing, and thorough research into methods of producing botulinum toxin is ongoing to meet such demand.

Currently, a representative commercially available product is Allergan's BOTOX^{®} (a botulinum toxin type A purified neurotoxin complex), and a 100-unit vial of BOTOX^{®} contains about 5 ng of purified botulinum toxin type A complex, 0.5 mg of human serum albumin, and 0.9 mg of sodium chloride and is provided in a vacuum-dried form and reconstituted using sterile saline without any preservative (injection of 0.9% sodium chloride). Other commercially available products include Dysport^{®} from Ipsen, UK (a Clostridium botulinum type A toxin hemagglutinin complex with lactose and human serum albumin in a botulinum toxin pharmaceutical composition, reconstituted with 0.9% sodium chloride before use) and MyoBloc^{®} from Solstice Neurosciences (an injectable solution containing botulinum toxin type B, human serum albumin, sodium succinate, and sodium chloride, about pH 5.6).

Botulinum toxin is mainly distributed as a freeze-dried formulation, and many studies have been conducted to keep the freeze-dried formulation stable for a long period of time. For example, US 7,744,904 B1 attempted to improve the stability of botulinum toxin by forming a cyclodextrin-botulinum toxin complex through simple mixing of alpha, beta, and gamma cyclodextrins with botulinum toxin in phosphate-buffered saline. In case of alpha, beta, and gamma cyclodextrins, however, oral administration is allowed, but there are problems in that they are not suitable as an excipient for botulinum toxin, which is mainly used as an injection, due to limited solubility and renal toxicity thereof. Moreover, when botulinum toxin having a non-uniform particle size is injected into the body, it does not work properly in the body, so that desired therapeutic or cosmetic effects may not be achieved, causing various side effects.

Therefore, the present inventors have made efforts to develop a freeze-dried formulation in which botulinum toxin is homogeneously granulated and stability is also enhanced, and thus ascertained that, when a botulinum toxin freeze-dried formulation containing protamine sulfate as an excipient is prepared, protamine sulfate and botulinum toxin form a complex, so that the efficacy of botulinum toxin may last for 6 months or longer compared to the previous 3 months, and botulinum toxin particles may be uniformly prepared with a PDI value of 0.3 or less, thus culminating in the present invention.

### Citation List

### Patent Literature

(Patent Document 1) US 7,744,904 B1

### [Disclosure]

It is an object of the present invention to provide a novel botulinum toxin freeze-dried formulation composition with a uniform botulinum toxin particle size and improved long-term storage stability, and a method of preparing the same.

In order to accomplish the above object, the present invention provides a botulinum toxin freeze-dried formulation composition including a complex of botulinum toxin and protamine sulfate, an isotonic agent, and a buffering agent.

In addition, the present invention provides a method of preparing a botulinum toxin freeze-dried formulation, including:
(a) forming a complex of botulinum toxin and protamine sulfate by mixing botulinum toxin and protamine sulfate;
(b) preparing a botulinum toxin stock solution by adding at least one excipient selected from the group consisting of polysorbate 20, hydroxypropyl-beta-cyclodextrin, human serum albumin, and mannitol, an isotonic agent, and a buffering agent to the complex of botulinum toxin and protamine sulfate; and
(c) freeze-drying the botulinum toxin stock solution.

### [Mode for Invention]

Unless otherwise defined, all technical and scientific terms used herein have the same meanings as typically understood by those skilled in the art to which the present invention belongs. In general, the nomenclature used herein is well known in the art and is typical.

In the present invention, it was confirmed that, when a complex of botulinum toxin and protamine sulfate is prepared and formulated in a freeze-dried form, long-term storage stability of the freeze-dried formulation may be improved while uniformly maintaining the size thereof.

Accordingly, an aspect of the present invention pertains to a botulinum toxin freeze-dried formulation composition including a complex of botulinum toxin and protamine sulfate, an isotonic agent, and a buffering agent.

In the present invention, botulinum toxin that is negatively charged and protamine sulfate that is positively charged form an ionic complex when reacting in a solution, which is particularly configured such that protamine sulfate binds to a high-molecular-weight botulinum toxin protein through ionic bonding. As such, the amount of protamine sulfate binding to botulinum toxin may vary depending on the concentration of protamine sulfate that is added, and the surface of botulinum toxin to which protamine sulfate binds may be positively charged, forming an additional complex with botulinum toxin.

Therefore, in order to form a complex with improved long-term storage stability of the freeze-dried formulation according to the present invention, the concentration of protamine sulfate in the present invention may be 1.72 nM to 0.22 mM, preferably 1.72 nM to 17.2 nM, more preferably 1.72 nM to 6.98 nM, much more preferably 1.72 nM to 3.44 nM, but is not limited thereto.

Also, in the present invention, the botulinum toxin and protamine sulfate may form a complex in a mass ratio of 1:3 to 1:20, preferably 1:5 to 1:20, more preferably 1:5 to 1:10, most preferably 1:5, but the present invention is not limited thereto.

In the present invention, the composition may further include at least one excipient selected from the group consisting of polysorbate 20, hydroxypropyl-beta-cyclodextrin, human serum albumin, and mannitol, and preferably further includes hydroxypropyl-beta-cyclodextrin, human serum albumin, and mannitol, but the present invention is not limited thereto.

In the present invention, the concentration of the hydroxypropyl-beta-cyclodextrin may be 1 to 30 w/v%, preferably 2 to 25 w/v%, more preferably 5 to 20 w/v%, for example, 5 to 15 w/v%, 5 to 10 w/v%, 8 to 15 w/v%, 8 to 10 w/v%, 10 to 20 w/v%, or 15 to 20 w/v%, but is not limited thereto.

In the present invention, the concentration of human serum albumin may be 0.1 to 1.0 w/v%, preferably 0.2 to 0.7 w/v%, more preferably 0.2 to 0.5 w/v%, most preferably 0.35 to 0.5 w/v%, but is not limited thereto.

In the present invention, mannitol functions as a cryoprotectant, and sucrose, glycerin, trehalose, or lactose may be used instead thereof, but the present invention is not limited thereto. The amount of the cryoprotectant may be 0.1 to 8 w/v% based on the total amount of the composition, but is not limited thereto.

In the present invention, the cryoprotectant is preferably mannitol, and the concentration of mannitol may be 0.1 to 10 w/v%, preferably 0.1 to 8 w/v%, more preferably 4 to 8 w/v%, but is not limited thereto.

In the present invention, the isotonic agent used may be sodium chloride, glycerin, mannitol, sucrose, potassium chloride, or dextrose, but is not limited thereto. The amount of the isotonic agent may be 0.7 to 0.95 w/v% based on the total amount of the composition, but is not limited thereto.

In the present invention, the isotonic agent is preferably sodium chloride, and the concentration of sodium chloride may be 0.7 to 1.0 w/v%, preferably 0.7 to 0.95 w/v%, more preferably 0.8 to 0.95 w/v%, most preferably 0.9 w/v%, but is not limited thereto.

The composition according to the present invention may contain a buffering agent that is physiologically compatible showing a pH equal to or higher than an isoelectric point, thereby ensuring long-term stability.

In the present invention, the pH of the buffering agent that is physiologically compatible has to be maintained in the range of 6.0 to 7.0, preferably 6.4 to 6.6, and most preferably a buffering agent that results in a pH of about 6.5 is used.

In the present invention, the physiologically compatible buffering agent used may be sodium citrate, succinic acid, phosphoric acid, monobasic potassium phosphate, sodium acetate, or sodium chloride, but is not limited thereto.

In the present invention, the concentration of the buffering agent suitable for freeze-drying may be 10 to 35 mM, preferably 10 to 30 mM, most preferably 15 to 25 mM based on the total concentration of the composition.

Preferably, the buffering agent has a pH of 6.0 to 7.0 and a concentration of 10 to 35 mM, but the present invention is not limited thereto.

In an embodiment, as the physiologically compatible buffering agent, phosphoric acid having a pH of 6.5 may be contained at a concentration of 20 mM based on the total concentration of the composition.

The freeze-dried formulation composition according to the present invention is imparted with hydrophobicity by forming a complex of botulinum toxin and protamine sulfate and then adding hydroxypropyl-beta-cyclodextrin and human-derived serum albumin thereto. Also, the solubility of botulinum toxin may be increased using an isotonic agent, preferably sodium chloride.

In the present invention, the protamine sulfate is a molecule containing a large amount of positively charged amino acid, and forms a complex with botulinum toxin, contributing to the stability of botulinum toxin.

In the present invention, the hydroxypropyl-beta-cyclodextrin (which may be used interchangeably with 'Betadex') has a sugar chain having a cyclic structure and has amphipathic properties similar to polysorbate 20 or polysorbate 80, contributing to the stability of botulinum toxin.

In the present invention, "botulinum toxin" may include not only neurotoxins (NTXs) produced by *Clostridium botulinum* strains or variants thereof, but also modified, recombinant, hybrid, and chimeric botulinum toxins. In the present invention, recombinant botulinum toxin may have a light chain and/or a heavy chain recombinantly produced by non-Clostridium species. In the present invention, the botulinum toxin includes pure botulinum toxin (i.e. about 150 kDa neurotoxic molecule), as well as botulinum toxin complexes (i.e. 300, 600, and 900 kDa complexes).

In the present invention, the botulinum toxin may be selected from the group consisting of serotypes A, B, C, D, E, F, and G, but is not limited thereto.

Preferably, the botulinum toxin contained in the freeze-dried composition of the present invention is botulinum toxin type A. The composition according to the present invention may contain about 10 to 200 units/ml, preferably about 20 to 150 units/ml, more preferably about 40 to 100 units/ml of botulinum toxin, but the present invention is limited thereto.

In the present invention, the freeze-dried composition may further contain an antioxidant, and the antioxidant may be alpha-tocopherol, but is not limited thereto.

Another aspect of the present invention pertains to a method of preparing a botulinum toxin freeze-dried formulation, including:
(a) forming a complex of botulinum toxin and protamine sulfate by mixing botulinum toxin and protamine sulfate;
(b) preparing a botulinum toxin stock solution by adding at least one excipient selected from the group consisting of polysorbate 20, hydroxypropyl-beta-cyclodextrin, human serum albumin, and mannitol, an isotonic agent, and a buffering agent to the complex of botulinum toxin and protamine sulfate; and
(c) freeze-drying the botulinum toxin stock solution.

A better understanding of the present invention may be obtained through the following examples. These examples are merely set forth to illustrate the present invention, and are not to be construed as limiting the scope of the present invention, as will be obvious to those skilled in the art.

### Experimental Example 1. Preparation of botulinum toxin/protamine sulfate complex and measurement of size thereof

In order to maintain the stability of botulinum toxin, a botulinum toxin/protamine sulfate complex was prepared using protamine sulfate that is positively charged. The relatively negatively charged botulinum toxin used a buffer solution at a pH of 6 or higher to form a complex with protamine sulfate at a pH of about 6. 20 mM phosphate-buffered saline (pH 6.5) was used as the buffer solution. Botulinum toxin type A (900 kDa, Daewoong Co., Ltd.) was prepared at 0.1 mg/ml, and protamine sulfate was dissolved at 0.5, 1.0, and 2.0 mg/ml in 20 mM phosphate-buffered saline (pH 6.5), followed by mixing at 1:1 (v/v) and then complex formation at room temperature for about 30 minutes.

In order to confirm whether or not the complex was formed, the particle size of the botulinum toxin or the botulinum toxin/protamine sulfate complex was measured using a nanoparticle size analyzer (Zetasizer ZSP, Malvern). The nanoparticle size analyzer is a device that measures the particle size by applying light to a sample and measuring a refractive index when the light is reflected therefrom. Based on results of the test, as shown in Table 1 below, the size of botulinum toxin type A was determined to be about 19 nm and the size of the botulinum toxin/protamine sulfate complex at a ratio of 1:5 was determined to be about 182 nm. At 1:10 and 1:20, respective sizes thereof were determined to be about 335 and 481 nm. Also, based on results of measurement of polydispersity index (PDI), when the complex of botulinum toxin type A and protamine sulfate at a ratio of 1:5 was formed, the PDI value thereof was 0.3 or less, indicating that the complex at the corresponding ratio had a uniform particle size. Therefore, the above composition ratio was found to be the most advantageous for individual complexes to exert the same effect when injected into the body.

**[Table 1]**

| Measurement of size of botulinum toxin/protamine sulfate complex | | |
|---|---|---|
| **Sample** | **size (nm)** | **PDI** |
| Botulinum toxin type A | 18.58±5.12 | 0.258 |
| Botulinum toxin type A/protamine sulfate 1:5 complex | 182±51.9 | 0.269 |
| Botulinum toxin type A/protamine sulfate 1:10 complex | 334.7±74.8 | 0.391 |
| Botulinum toxin type A/protamine sulfate 1:20 complex | 481.4±79.1 | 0.488 |

### Experimental Example 2. Screening of excipient in freeze-dried formulation composition including botulinum toxin as main ingredient and evaluation of stability under severe conditions

For the botulinum toxin freeze-dried formulation including the botulinum toxin/protamine sulfate 1:5 complex as a basic composition, an excipient composition having stability was screened. In the freeze-dried formulation composition including the botulinum toxin/protamine sulfate complex using 20 mM phosphate-buffered saline (pH 6.5) as a basic solvent, various excipients such as stabilizers, antioxidants, cryoprotectants, and isotonic agents were screened and the stability of botulinum toxin was confirmed.

As such, an experiment was conducted with 40 U/ml of botulinum toxin, and a final stock solution with the excipient composition shown in Table 2 below was prepared, dispensed at 50 U/vial, and freeze-dried. After freeze-drying, a stability test under severe conditions was performed to indirectly confirm long-term stability. The samples were stored for 12 weeks in a chamber under severe conditions at 40°C, and toxin storage stability was confirmed by measuring the change in titer. Specifically, the stability of botulinum toxin was evaluated through an animal titer test using 4-week-old ICR mice (Koatech, Korea) for the indicated specific period, and the test material was rehydrated with saline and then diluted to seven concentrations, the diluted solution was administered at 0.1 ml/mouse, the death of animals was observed for 3 days, and the LD50 value was determined to calculate the titer value. The experiment was performed with 10 mice at each concentration, and the results thereof are represented as the average value thereof.

Here, the titer of each formulation was set to 100% upon preparation. However, since botulinum toxin is a protein and is affected by the composition of excipients, external environment, freeze-drying parameters, and the like, the titer was converted again to 100% on the first day under severe conditions, and the change in titer was then calculated. As such, the titer was judged to be suitable up to ±20% based on 100% of the initial titer value.

**[Table 2]**

| Classificati on | Stabilizer | | Antioxidant | | Isotonic agent | | Cryoprotecta nt | | Sever e 4 weeks (40°C ) | Sever e 12 weeks (40°C ) | Titer |
|---|---|---|---|---|---|---|---|---|---|---|---|
| | Material | Conc (%) | Material | Conc (%) | Materi al | Conc (%) | Materi al | Conc (%) | | | |
| C.Ex.1 | HSA | 0.5 | - | - | - | - | Mannit ol | 5 | 0 | 0 | Unsuitab le |
| C.Ex.2 | Betadex | 5 | - | - | Sodium chlori de | 0.9 | - | - | 0 | 0 | Unsuitab le |
| | HSA | 0.5 | | | | | | | | | |
| Ex.1 | Betadex | 5 | - | - | Sodium chlori de | 0. 9 | Mannit ol | 5 | 121.2 | 105.4 | Suitable |
| | HSA | 0.5 | | | | | | | | | |
| Ex.2 | Polysorba te 20 | 0.01 | - | - | Sodium chlori de | 0. 9 | Mannit ol | 5 | 95.3 | 90. 6 | Suitable |
| | Betadex | 5 | | | | | | | | | |
| | HSA | 0.5 | | | | | | | | | |
| Ex.3 | Polysorba te 20 | 0.01 | α-Tocopher ol | 0.03 5 | Sodium chlori de | 0. 9 | Mannit ol | 5 | 120.2 | 105.4 | Suitable |
| | Betadex | 5 | | | | | | | | | |
| | HSA | 0.5 | | | | | | | | | |

Thereby, when human serum albumin (HSA) as a stabilizer and mannitol as a cryoprotectant were used (Comparative Example 1) and when Betadex and human serum albumin as stabilizers and sodium chloride as an isotonic agent were used (Comparative Example 2), both Comparative Examples 1 and 2 were judged to be unsuitable based on results of measurement of the titer of botulinum toxin after 12 weeks under severe conditions. However, when Betadex and human serum albumin as stabilizers, sodium chloride as an isotonic agent, and mannitol as a cryoprotectant were used (Example 1), the titer of botulinum toxin was confirmed to be the best after 12 weeks under severe conditions. Also, when polysorbate, human serum albumin, and Betadex as stabilizers, sodium chloride as an isotonic agent, and mannitol as a cryoprotectant were used (Example 2) and when alpha-tocopherol as an antioxidant was further added thereto (Example 3), the titer after 12 weeks under severe conditions was judged to be suitable. However, taking into consideration the above experimental results and the economic costs of excipients, Example 1 was selected as the basic excipient composition and further studies were carried out.

### Experimental example 3. Optimization of excipient composition and evaluation of stability under severe conditions

The botulinum toxin/protamine sulfate 1:5 complex was used as a basic composition and 0.9% sodium chloride and 20 mM phosphate-buffered saline (pH 6.5) were fixed, and in order to confirm the effect in the concentration ranges containing 0.35-0.5 (w/v)% of human serum albumin, 5-20 (w/v)% of Betadex, and 4-8 (w/v)% of mannitol, three additional compositions were prepared as shown in Table 3 below. All compositions were prepared in the same manner except for the concentration of the excipient. After preparation, the compositions were dispensed into 10 ml vials and freeze-dried, and the stability of botulinum toxin was evaluated under severe conditions for 12 weeks.

**[Table 3]**

| Batch No. | HSA | HP Betadex | D-Mannintol | Severe 2 weeks (40°C) | Severe 6 weeks (40°C) | Severe 12 weeks (40°C) |
|---|---|---|---|---|---|---|
| Example 1 | 0.5 | 5 | 5 | 124 | 121.2 (Severe 4 weeks) | 105.4 |
| Example 4 | 0.5 | 10 | 4 | 149.3 | 157.8 | 103.1 |
| Example 5 | 0.35 | 20 | 4 | 101 | 111.1 | 89.1 |
| Example 6 | 0.5 | 20 | 8 | 134.1 | 115.8 | 94.7 |

Thereby, it was experimentally verified that the stability of botulinum toxin was maintained for 8 weeks or more, particularly 12 weeks under severe conditions in the concentration ranges containing 0.35-0.5% of human serum albumin, 5-20% of Betadex, and 4-8% of mannitol. Based on the results of conventional studies, the present inventors have confirmed that stability may be maintained for up to about 6 months under refrigeration when ensuring stability for 2 weeks under severe conditions and stability may be maintained for about 2 years or longer under refrigeration when ensuring stability for 8 weeks under severe conditions. Therefore, the efficacy of the botulinum toxin freeze-dried formulation containing 0.35-0.5% of human serum albumin, 10-20% of Betadex, and 4-8% of mannitol, as an optimal composition with stability within the titer range of 80 to 120% up to 12 weeks under severe conditions, was found to be maintained for at least 6 months, preferably 12 months or longer, most preferably 24 months or longer during distribution.

### Experimental Example 4. Evaluation of effect of cyclodextrin-based excipient on stability of botulinum toxin

Betadex is a derivative of beta (β)-cyclodextrin and is a material with improved hydrophilic properties by introducing a hydroxyl functional group and a propyl functional group. Betadex is currently an excipient approved by the FDA, EU, etc. as a pharmaceutical excipient and is used as a main ingredient stabilizer of pharmaceuticals. Cyclodextrins are classified into three types of alpha (α), beta (β), and gamma (γ) depending on structures thereof, and the internal structure with relatively hydrophobic properties is stabilized through inclusion of the main ingredient. Therefore, the effect of cyclodextrin-based excipients similar to Betadex on stability of botulinum toxin was evaluated.

Here, since α, β, and γ cyclodextrins have renal toxicity and are evaluated to be unsuitable as excipients for pharmaceutical injections by the FDA and KFDA, they were excluded from this experiment, and whether methyl-beta-cyclodextrin, beta-cyclodextrin sulfate, beta-cyclodextrin sulfobutyl ether sodium, hydroxyethyl-beta-cyclodextrin, hydroxypropyl-gamma-cyclodextrin, or hydroxypropyl-alpha-cyclodextrin could be used instead of Betadex (hydroxypropyl-beta-cyclodextrin) was tested.

To this end, the botulinum toxin/protamine sulfate 1:5 complex was used as a basic composition and 0.9% sodium chloride, 20 mM phosphate-buffered saline (pH 6.5), 0.5% human serum albumin, and 4% mannitol were fixed, and only cyclodextrin-based excipients were changed, and an experiment was conducted. Here, the concentration of the cyclodextrin-based excipient was set to 5%.

**[Table 4]**

| Batch No. | Cyclodextrin-based excipient | Severe 2 weeks (40°C) | Severe 4 weeks (40°C) | Severe 6 weeks (40°C) |
|---|---|---|---|---|
| Comparative Example 3 | Methyl-beta-cyclodextrin | 105.4 | 76.4 | - |
| Comparative Example 4 | Beta-cyclodextrin sulfate | 112.5 | 73.7 | - |
| Comparative Example 5 | Beta-cyclodextrin sulfobutyl ether sodium | 92.4 | 61.5 | - |
| Comparative Example 6 | Hydroxyethyl-beta-cyclodextrin | | | |
| Comparative Example 7 | Hydroxypropyl-gamma-cyclodextrin | 66.9 | - | - |
| Comparative Example 8 | Hydroxypropyl-alpha-cyclodextrin | 63.4 | - | - |
| Example 7 | Hydroxypropyl-beta-cyclodextrin | 81.4 | 84.7 | 104.8 |

Thereby, as shown in Table 4, when using other cyclodextrin-based excipients, except for Betadex (hydroxypropyl-beta-cyclodextrin, Example 7) according to the present invention, the storage stability of botulinum toxin for 4 weeks under severe conditions was determined to be unsuitable, and titer stability was not maintained for 6 weeks or more under severe conditions. Therefore, it was found that the use of Betadex among cyclodextrins was very effective in the formulation for cryopreservation of botulinum toxin according to the present invention.

### Experimental Example 5. Evaluation of effect of protamine sulfate on stability of botulinum toxin

Protamine sulfate that is a positively charged peptide is capable of forming a complex with relatively negatively charged botulinum toxin through ionic bonding. Complex formation with botulinum toxin may act as a factor for stably maintaining a botulinum toxin protein. Accordingly, a comparative test was performed to confirm the effect of the presence/absence of protamine sulfate on the stability of botulinum toxin.

To this end, 0.9% sodium chloride, 20 mM phosphate-buffered saline (pH 6.5), 0.5% human serum albumin, 4% mannitol, and 10% Betadex were fixed, and botulinum toxin alone or the botulinum toxin/protamine sulfate 1:5 complex was used, and a comparative experiment was performed.

**[Table 5]**

| Batch No. | Protamine sulfate (ng/ml) | Severe 1 week (40°C) | Severe 3 weeks (40°C) | Severe 5 weeks (40°C) | Severe 8 weeks (40°C) | Severe 10 weeks (40°C) |
|---|---|---|---|---|---|---|
| Comparative Example 9 | 0 | 77.4 | 62.8 | - | - | - |
| Example 4 | 7.75 | 125.4 | 110.1 | 87.1 | 89 | 89.9 |

Thereby, as shown in Table 5, it was confirmed that the stability of the botulinum toxin freeze-dried formulation was maintained only in the formulation including protamine sulfate.

Having described specific parts of the present invention in detail above, it will be obvious to those skilled in the art that these specific descriptions are only preferred embodiments, and the scope of the present invention is not limited thereby. Accordingly, the substantial scope of the present invention will be defined by the appended claims.

### [Industrial Applicability]

According to the present invention, a botulinum toxin freeze-dried formulation is capable of reducing the inactivation rate of botulinum toxin during a freeze-drying process, yielding an efficient and stable freeze-dried formulation. In addition, the botulinum toxin freeze-dried formulation according to the present invention can maintain the stability of botulinum toxin for a long period of time compared to conventional botulinum toxin freeze-dried formulations, and also has a consistent effect at the site of administration due to formation of a uniform particle size and can prevent side effects that cause excessive toxin administration effects only at some sites.

## Claims

1. A botulinum toxin freeze-dried formulation composition comprising a complex of botulinum toxin and protamine sulfate, an isotonic agent, and a buffering agent.

2. The botulinum toxin freeze-dried formulation composition according to claim 1, wherein a concentration of the protamine sulfate is 1.72 nM to 0.22 mM.

3. The botulinum toxin freeze-dried formulation composition according to claim 1, wherein the botulinum toxin and the protamine sulfate form a complex in a mass ratio of 1:5 to 1:20.

4. The botulinum toxin freeze-dried formulation composition according to claim 1, wherein the composition further comprises at least one excipient selected from the group consisting of polysorbate 20, hydroxypropyl-beta-cyclodextrin, human serum albumin, and mannitol.

5. The botulinum toxin freeze-dried formulation composition according to claim 4, wherein the composition further comprises hydroxypropyl-beta-cyclodextrin, human serum albumin, and mannitol.

6. The botulinum toxin freeze-dried formulation composition according to claim 5, wherein a concentration of the hydroxypropyl-beta-cyclodextrin is 5 to 20 w/v%.

7. The botulinum toxin freeze-dried formulation composition according to claim 5, wherein a concentration of the human serum albumin is 0.35 to 0.5 w/v%.

8. The botulinum toxin freeze-dried formulation composition according to claim 5, wherein a concentration of the mannitol is 4 to 8 w/v%.

9. The botulinum toxin freeze-dried formulation composition according to claim 1, wherein a concentration of the isotonic agent is 0.7 to 1.0 w/v%.

10. The botulinum toxin freeze-dried formulation composition according to claim 1, wherein the buffering agent has a pH of 6.0 to 7.0 and a concentration of 10 to 35 mM.

11. The botulinum toxin freeze-dried formulation composition according to claim 1, wherein the botulinum toxin is selected from the group consisting of serotypes A, B, C, D, E, F, and G.

12. A method of preparing a botulinum toxin freeze-dried formulation, comprising:
(a) forming a complex of botulinum toxin and protamine sulfate by mixing botulinum toxin and protamine sulfate;
(b) preparing a botulinum toxin stock solution by adding at least one excipient selected from the group consisting of polysorbate 20, hydroxypropyl-beta-cyclodextrin, human serum albumin, and mannitol, an isotonic agent, and a buffering agent to the complex of botulinum toxin and protamine sulfate; and
(c) freeze-drying the botulinum toxin stock solution.

## Patentansprüche

1. Gefriergetrocknete Botulinumtoxin-Formulierungszusammensetzung, die einen Komplex aus Botulinumtoxin und Protaminsulfat, einem isotonischen Mittel und einem Puffermittel umfasst.

2. Gefriergetrocknete Botulinumtoxin-Formulierungszusammensetzung nach Anspruch 1, wobei die Konzentration des Protaminsulfats 1,72 nM bis 0,22 mM beträgt.

3. Gefriergetrocknete Botulinumtoxin-Formulierungszusammensetzung nach Anspruch 1, wobei das Botulinumtoxin und das Protaminsulfat einen Komplex in einem Massenverhältnis von 1:5 bis 1:20 bilden.

4. Gefriergetrocknete Botulinumtoxin-Formulierungszusammensetzung nach Anspruch 1, wobei die Zusammensetzung weiters zumindest einen Hilfsstoff umfasst, der aus der aus Polysorbat 20, Hydroxypropyl-β-cyclodextrin, menschlichem Serumalbumin und Mannit bestehenden Gruppe ausgewählt ist.

5. Gefriergetrocknete Botulinumtoxin-Formulierungszusammensetzung nach Anspruch 4, wobei die Zusammensetzung weiters Hydroxypropyl-β-cyclodextrin, menschliches Serumalbumin und Mannit umfasst.

6. Gefriergetrocknete Botulinumtoxin-Formulierungszusammensetzung nach Anspruch 5, wobei die Konzentration des Hydroxypropyl-β-cyclodextrins 5 bis 20 % (Gew./Vol.) beträgt.

7. Gefriergetrocknete Botulinumtoxin-Formulierungszusammensetzung nach Anspruch 5, wobei die Konzentration des menschlichen Serumalbumins 0,35 % bis 0,5 % (Gew./Vol.) beträgt.

8. Gefriergetrocknete Botulinumtoxin-Formulierungszusammensetzung nach Anspruch 5, wobei die Konzentration des Mannits 4 % bis 8 % (Gew./Vol.) beträgt.

9. Gefriergetrocknete Botulinumtoxin-Formulierungszusammensetzung nach Anspruch 1, wobei die Konzentration des isotonischen Mittels 0,7 % bis 1,0 % (Gew./Vol.) beträgt.

10. Gefriergetrocknete Botulinumtoxin-Formulierungszusammensetzung nach Anspruch 1, wobei das Puffermittel einen pH-Wert von 6,0 bis 7,0 und eine Konzentration von 10 bis 35 mM aufweist.

11. Gefriergetrocknete Botulinumtoxin-Formulierungszusammensetzung nach Anspruch 1, wobei das Botulinumtoxin aus der aus den Serotypen A, B, C, D, E, F und G bestehenden Gruppe ausgewählt ist.

12. Verfahren zur Herstellung einer gefriergetrockneten Botulinumtoxin-Formulierungszusammensetzung, das Folgendes umfasst:
(a) das Bilden eines Komplexes aus Botulinumtoxin und Protaminsulfat durch Vermischen von Botulinumtoxin und Protaminsulfat;
(b) das Herstellen einer Botulinumtoxin-Stammlösung durch Zusetzen von zumindest einem Hilfsstoff, der aus der aus Polysorbat 20, Hydroxypropyl-beta-cyclodextrin, menschlichem Serumalbumin und Mannit bestehenden Gruppe ausgewählt ist, eines isotonischen Mittels und eines Puffermittels zu dem Komplex aus Botulinumtoxin und Protaminsulfat; und
(c) das Gefriertrocknen der Botulinumtoxin-Stammlösung.

## Revendications

1. Composition de formulation lyophilisée à base de toxine botulique comprenant un complexe de toxine botulique et de sulfate de protamine, un agent isotonique et un agent tampon.

2. Composition de formulation lyophilisée de toxine botulique selon la revendication 1, dans laquelle une concentration du sulfate de protamine est de 1,72 nM à 0,22 mM.

3. Composition de formulation lyophilisée de toxine botulique selon la revendication 1, dans laquelle la toxine botulique et le sulfate de protamine forment un complexe selon un rapport massique de 1:5 à 1:20.

4. Composition de formulation lyophilisée de toxine botulique selon la revendication 1, dans laquelle la composition comprend en outre au moins un excipient choisi dans le groupe constitué du polysorbate 20, de l'hydroxypropyl-bêta-cyclodextrine, de l'albumine sérique humaine et du mannitol.

5. Composition de formulation lyophilisée de toxine botulique selon la revendication 4, dans laquelle la composition comprend en outre de l'hydroxypropyl-bêta-cyclodextrine, de l'albumine sérique humaine et du mannitol.

6. Composition de formulation lyophilisée de toxine botulique selon la revendication 5, dans laquelle une concentration de l'hydroxypropyl-bêta-cyclodextrine est de 5 à 20 % en poids/volume.

7. Composition de formulation lyophilisée de toxine botulique selon la revendication 5, dans laquelle une concentration de l'albumine sérique humaine est de 0,35 à 0,5 % en poids/volume.

8. Composition de formulation lyophilisée de toxine botulique selon la revendication 5, dans laquelle une concentration du mannitol est de 4 à 8 % en poids/volume.

9. Composition de formulation lyophilisée de toxine botulinique selon la revendication 1, dans laquelle une concentration de l'agent isotonique est de 0,7 à 1,0 % en poids/volume.

10. Composition de formulation lyophilisée de toxine botulique selon la revendication 1, dans laquelle l'agent tampon présente un pH de 6,0 à 7,0 et une concentration de 10 à 35 mM.

11. Composition de formulation lyophilisée de toxine botulique selon la revendication 1, dans laquelle la toxine botulique est choisie dans le groupe comprenant les sérotypes A, B, C, D, E, F et G.

12. Procédé de préparation d'une formulation lyophilisée de toxine botulique, comprenant les étapes consistant à :
(a) former un complexe de toxine botulique et de sulfate de protamine en mélangeant la toxine botulique et le sulfate de protamine ;
(b) préparer une solution mère de toxine botulique en ajoutant au moins un excipient choisi dans le groupe constitué de polysorbate 20, d'hydroxypropyl-bêta-cyclodextrine, d'albumine sérique humaine et de mannitol, un agent isotonique et un agent tampon au complexe de toxine botulique et de sulfate de protamine ; et
(c) lyophiliser la solution mère de toxine botulique.
